# FASCICULE DE BREVET EUROPEEN

(11) **EP 1 047 395 B1**
(45) Date de publication et mention de la délivrance du brevet: **06.05.2004**
(21) Numéro de dépôt: 99900497.1
(22) Date de dépôt: 06.01.1999
(51) Int. Cl.: A61K 7/48

(54) **COMPOSITION COMPRENANT DU MIEL, AU MOINS UNE HUILE ESSENTIELLE ET/OU AU MOINS UN DERIVE D'UNE HUILE ESSENTIELLE**
HONIG, MINDESTENS EIN ÄTHERISCHES ÖL UND/ODER EIN DERIVAT EINES ÄTHERISCHEN ÖLS ENTHALTENDE ZUSAMMENSETZUNG
MIXTURE CONTAINING HONEY, AT LEAST ONE ESSENTIAL OIL AND/OR AT LEAST ONE ESSENTIAL OIL DERIVATIVE

(30) Priorité: 16.01.1998 FR 9800451
(43) Date de publication de la demande: 02.11.2000
(73) Titulaire: Morice, André Pierre, 56100 Lorient (FR)
(72) Inventeur: Morice, André Pierre, 56100 Lorient (FR)
(74) Mandataire: Ballot, Paul
(86) Numéro de dépôt international: PCT/FR1999/000010
(87) Numéro de publication internationale: WO 1999/036052

(56) Documents cités:
- FR-A- 2 727 627
- BASE DE DONN ES "CHEMICAL ABSTRACTS" (SERVEUR: STN); abrégé 103: 27 321, Colombus, OH, USA; & RO 82 337 B (INTREPRINDERA PENTRU OMOGENIZAREA MIERII SI PRODUCTIE ALIMENTARA) 30 SEPTEMBRE 1983 XP002097019
- BASE DE DONN ES "CHEMICAL ABSTRACTS" (SERVEUR: STN); abrégé 126: 18 147, Colombus, OH, USA; & LI 3104 B (V. SEMENIENE et al.) 25 NOVEMBRE 1994 XP002097020
- BASE DE DONN ES "CHEMICAL ABSTRACTS" (SERVEUR: STN); abrégé 103: 177 287, Colombus, OH, USA; & HU 35 165 O(J. MATHE et al.) 28 JUIN 1985 XP002097021

## Description

La présente invention se rapporte à une composition comprenant du miel.

Le miel mondialement connu pour ses origines diverses, est déjà utilisé, seul, pour ses propriétés régénératrices cellulaires et/ou tissulaires.

Le miel présente l'inconvénient de présenter différents germes en une quantité fonction de son origine. Par conséquent, bien que présentant en outre des caractéristiques nutritives et gustatives intéressantes, le miel ne peut pas être utilisé en application médicale puisqu'il ne constitue pas un milieu stérile.

Par ailleurs, les huiles essentielles sont déjà utilisées dans l'industrie des parfums et dans celle de l'alimentaire pour leurs propriétés olfatives et/ou gustatives.

Aussi il subsiste un besoin de disposer d'une composition stérile, présentant des caractéristiques nutritives et gustatives intéressantes, et pouvant être utilisée sans risque pour l'organisme humain et/ou animal dans le domaine thérapeutique et/ou cosmétique et/ou alimentaire.

Le document Chemical Abstract 103: 27 321 décrit une composition pharmaceutique dont le coeur contient du miel et l'enrobage de l'huile de sariette.

La présente invention a pour objet une composition comprenant du miel, qui se caractérise en ce qu'elle comprend de 1 à 5% en poids d'au moins une huile essentielle et/ou au moins dérivé d'une huile essentielle choisie parmi le clou de girofle, la sarriette, l'origan, par rapport au poids total de la composition.

La composition selon l'invention présente l'avantage d'une part d'éliminer les germes pathogènes ou non, présents dans le miel, et permet d'autre part de fournir un support stérile, apte à contenir dans sa structure interne et/ou à recueillir sur sa surface, d'autres constituants et/ou molécules chimiques, à destination thérapeutique et/ou cosmétique et/ou alimentaire. La présence d'au moins une huile essentielle et/ou un dérivé d'une huile essentielle élimine les germes éventuellement présents dans le miel. La concentration des constituants chimiques ajoutés au support stérile est fonction de leurs natures, du seuil de tolérance de l'organisme pour chaque ingestion ou application cutanée et, de la structure physique du constituant chimique.

Ce support stérile présente, en outre, l'avantage d'avoir un goût sucré et agréable, facilitant ainsi son ingestion par de très jeunes enfants et/ou par des nourrissons.

La composition présente en outre l'intérêt d'utiliser comme constituant principal le miel qui est un produit d'origine naturelle et bon marché. Le miel, produit par les abeilles et récolté facilement dans les ruches, est exempt de substances exogènes, telles que des insecticides.

Par ailleurs, afin de pouvoir être utilisé dans la composition de l'invention, le miel, lors de la récolte, ne doit pas subir un quelconque traitement, tel que le chauffage, qui est souvent pratiqué par l'apiculteur pour faciliter son extraction de la ruche.

Parmi les affections du domaine thérapeutique pour lesquelles la composition peut être utilisée, on peut citer de façon non limitative, les affections O.R.L., telles que les rhumes, les grippes, les angines, les sinusites, en pneumologie la bronchite chronique, en gynécologie la ménopause, les bouffées de chaleur, en gastro-entérologie, la constipation, la diarrhée.

En dermatologie, la composition peut être utilisée dans les cas d'engelures, de verrues, de brûlures, de coups de soleil, d'acné et de cellulite.

Dans le domaine cosmétique, la composition peut notamment être utilisée dans des produits de traitement et/ou de soin de la peau tels que des crèmes hydratantes, anti-rides, amincissantes, dans des produits pour le nettoyage et/ou le soin du cuir chevelu tel que des shampoings, dans des savons, dans des produits pour les soins dentaires et buccaux tels que des pâtes dentifrices, dans des produits de thalassothérapie pour les bains.

Le miel est principalement constitué de 20% d'eau, 38% de lévulose, 31% de glucose, 1,3% de saccharose, 8,37% de composés acides (ayant un pH global de 3,91), 0,83% de protides et 0,5% de sels minéraux.

L'huile essentielle peut être choisie parmi les huiles essentielles naturelles ou synthétisées industriellement et leurs dérivés.

Pour obtenir la composition de l'invention, le miel est d'abord extrait des alvéoles disposées dans les hausses des ruches, par centrifugation, à température et pression ambiantes, puis il est mélangé avec au moins une huile essentielle avant qu'il ne cristallise.

De préférence, l'huile essentielle est choisie parmi les composés aromatiques et leurs esters, les flavonoïdes, les terpènes, les phénols, les alcools et leurs esters, les oxydes, les esters aliphatiques et cycliques, les composés aliphatiques sulfurés, les aldéhydes, les cétones, les lactones, les hétérocycles comprenant au moins un atome d'oxygène, les hétérocycles comprenant au moins un atome d'azote ou au moins un atome d'azote et un atome de soufre, les acides aromatiques et leurs esters, les esters aliphatiques et cycliques, et leurs dérivés.

La qualité d'une huile essentielle résulte des proportions de ses composants et de la présence de substances particulières souvent très faibles en quantité.

Parmi les huiles essentielles naturelles, la composition de l'invention peut notamment comprendre de l'Eucalyptus Radiata, de la Ravensara aromatica, de la Menthe, de la Lavande, du Thym, de la Sarriette, de la Sauge officinale, du Romarin, du Cèdre de l'atlantique, de la Girofle, du Vétiver, du Géranium, de la Bergamote, de la Coriandre, du Jasmin, de la Rose, et leurs dérivés.

Parmi les huiles essentielles obtenues par synthèse chimique, la composition peut comprendre des esters aliphatiques et cycliques tels que l'acétate de butyle (pomme), l'acétate d'isoamyle (banane), le butyrate d'éthyle (ananas), ou encore des cétones aliphatiques telles que le diacétyle (constituant du beurre), et leurs dérivés.

Parmi les alcools terpéniques acycliques, la composition peut comprendre le Géraniol (rose), le Linalol (muguet), et parmi les aldéhydes terpéniques acycliques, on peut utiliser le Citral (néral+géranial)
ou le Citronelall, et leurs dérivés.

Parmi les composés aliphatiques sulfurés, on peut utiliser le diallyldisulfure (ail), le cis et le transpropénylpropyldisulfure (oignon), ou encore parmi les alcools terpéniques cycliques et leurs esters, l'acétate de bornyle (aiguilles de pin), et leurs dérivés.

Parmi les cétones terpéniques cycliques, on peut utiliser la Menthone (menthe), la Carvone (odeur herbacée, et leurs dérivés). Dans les alcools aromatiques, on peut citer l'alcool benzylique, l'alcool trans-cinnamique (jacinthe), l'alcool 2-phényléthylène (rose, et leurs dérivés).

Parmi les aldéhydes aromatiques, on peut citer l'aldéhyde trans-cinnamique (cannelle), le benzaldéhyde (amande amère), ou encore parmi les acides aromatiques et leurs esters, l'acide phénylacétique (miel), le salicylate d'isoamyle (trèfle), l'anthranilate de méthyle (fleur d'oranger), et leurs dérivés.

Parmi les phénols et leurs esters et éthers, on peut utiliser le Thymol (thym), l'Eugénol (girofle), l'Anéthol (anis), et parmi les phénols aldéhydes, la Vanilline (vanille), et leurs dérivés.

Dans la famille des hétérocycles ayant au moins un oxygène, on peut utiliser la coumarine (foin), l'éthylmaltol (caramel), ou encore parmi les hétérocycles ayant au moins un atomes d'azote ou au moins un atome d'azote et de soufre, le 2-isobutylthiazole (tomate), et leurs dérivés.

La composition comprend du miel, au moins une huile essentielle et/ou au moins un dérivé d'une huile essentielle choisie parmi, le Clou de girofle, la sarriette des montagnes, l'Origan, et leurs dérivés.

De préférence, la composition comprend du miel et de l'Origan.

Le miel est de préférence compris en une quantité allant de 0,5 % à 25 % en poids , de préférence de 0,5% à 5% en poids, et plus particulièrement de 5% en poids par rapport au poids total de la composition.

L'huile essentielle est présente en une quantité allant de 1% à 5% en poids, de préférence de 1 à 2% en poids, et plus particulièrement de 1% en poids par rapport au poids total de la composition.

Plus particulièrement, la composition comprend 5% de miel et 1% d'Origan.

La composition selon l'invention peut comprendre de plus au moins un additif choisi parmi la propolis, les agents hydratants, les parfums, les vitamines, les oligo-éléments, les corps gras, les tensioactifs, les agents épaississants, les charges, les antioxygènes, les pigments, les gélifiants, les électrolytes, les protéines, les protides, les acides aminés, les glucides, les conservateurs, les colorants, l'eau, les alcools, la gelée royale, le pollen, les levures.

Parmi les oligo-éléments, on peut citer le potassium, le sélénium, le manganèse, le cobalt, le zinc, le cuivre, l'or, l'argent, le soufre. Parmi les vitamines qui peuvent être incorporées dans la composition de l'invention, on peut mentionner les vitamines A, B1, B2, B5, B6, B8, B12, C, D3, PP.

La propolis est principalement constituée de 50 à 55% de résine et de baume, 25 à 35% de cire, 10% d'huiles essentielles, 5% de pollen, 5% de diverses matières organiques et minérales.

La présence supplémentaire de propolis dans la composition de l'invention stimule l'effet antibactérien, antimycosique et antiviral de cette composition.

Selon une variante, la composition peut comprendre 5% de miel, 1% d'au moins une huile essentielle et/ou d'au moins un dérivé d'une huile essentielle, 1% de propolis.

L'additif est de préférence compris en une proportion allant de 80% à 95% en poids, de préférence de 85% à 88% en poids, et plus particulièrement de 90% en poids par rapport au poids total de la composition.

La composition de l'invention peut se présenter sous forme d'une émulsion huile dans eau ou eau dans huile, d'une solution aqueuse, d'une solution hydroalcoolique, de gel aqueux, de gel huileux, d'une dispersion de vésicules.

La composition selon l'invention peut se présenter sous différentes formes galéniques, telles que sous forme de suppositoire, de comprimé, de gélule, de pommade, d'ovule, de solution, de lotion, de crème, de lait, de shampoing, de savon, de rouge à lèvre, de fard, de fond de teint, de poudre, de dentifrice, de produit solaire, de déodorant, de spray, de pansement, de sirop, de collyre, de chewing gum, de boisson, de sucette, d'ampoules.

La composition de l'invention peut consister en une composition alimentaire, cosmétique et/ou de dermatologie pour la peau et/ou les muqueuses.

La composition de l'invention destinée à une utilisation alimentaire peut se présenter sous forme de produits anti-déprime, anti-stress, anti-vieillissement, de régime, de remise en forme, de produit aphrodisiaque, de produit contre l'insomnie.

Par ailleurs, cette composition peut être destinée à la désinfection des équipements sanitaires, tels que les salles de réanimation, les chambres de malades contagieux. La composition peut notamment se trouver sous forme d'aérosol combiné ou non à un désodorisant.

L'invention va maintenant être décrite à l'aide des exemples qui suivent et qui ne sont pas limitatifs.

Les proportions sont données en pourcentage pondéral.

### EXEMPLE 1 (non invention): Produit destiné au traitement des aphtes

- Miel 5%
- Hydrasol Origan 15%
- Hydrasol Ravensara 15%
- Hydrasol Myrte 15%
- Hydrasol Eucalyptus 50%

Cette composition, présente sous forme d'une solution pulvérisable, permet de faire disparaître totalement en quelques jours les aphtes présents sur les muqueuses de la paroi interne de la bouche, par une application biquotidienne.

### EXEMPLE 2 (non-invention): Produit destiné au traitement des affections mycosiques

- Miel 2%
- Propolis 10%
- Sauge 0,2%
- Sarriette 0,2%
- Origan 0,2%
- Thym 0,2%
- Lavande 0,2%
- Excipient (Lanoline) qsp 100

Cette composition, présente sous forme d'une pommade et, appliquée 3 fois par jour sur les lésions cutanées, permet la disparition complète en quelques jours des mycoses présentes sur la peau.

### EXEMPLE 3: Produit destiné au traitement de sinusite

- Miel 2%
- Thym blanc 1%
- Menthe 1%
- Eucalyptus 1%
- Pin sylvestre 1%
- Propolis 3%
- Parfum
- Excipient qsp 100%

Cette composition, présente sous forme d'une lotion inhalatrice, permet d'enrayer et de guérir une sinusite, en quelques jours.

### EXEMPLE 4 : Produit cicatrisant pour les plaies cutanées

Une première composition est appliquée sur les plaies. Cette composition est la suivante :
- Miel 5%
- Origan 1%
- Propolis 1%
- Parfum
- Excipient qsp 100

Dans le cas éventuel d'une surinfection de plaies, on applique sur la peau une seconde composition ayant pour formule :
- Miel 5%
- Origan 0,5%
- Sarriette 0,2%
- Sauge 0,3%
- Propolis 1%
- Excipient qsp 100

Ces compositions, présentes sous forme d'un pansement, permettent la cicatrisation des plaies en quelques jours suivant la profondeur des plaies.

### EXEMPLE 5 : Produit de soin et de nettoyage du cuir chevelu

- Miel 2%
- Sauge 0,5%
- Eucalyptus 0,5%
- Levure de bière 1%
- Tensioactif 1%
- Agent hydratant (glycérol) 2%
- Parfum
- Eau qsp 100

Cette composition, présente sous forme d'un shampoing antipelliculaire, permet la disparition des pellicules présentes sur la peau en quelques jours.

### EXEMPLE 6 : Produit destiné au traitement des infections présentes sur la parois internes du vagin

- Miel 0,5%
- Mélaleuca 0,5%
- Propolis 2%
- Excipient qsp 100

Ce produit, présent sous forme d'ovule, permet le traitement des infections en quelques jours par une prise quotidienne.

Les tableaux qui suivent montrent l'efficacité de la fonction antibactérienne de la composition de l'invention, en fonction de la nature de l'huile essentielle ou de dérivé d'huile essentielle utilisée, lorsque cette composition est mise en présence de souches de germes appartenant à des genres différents.

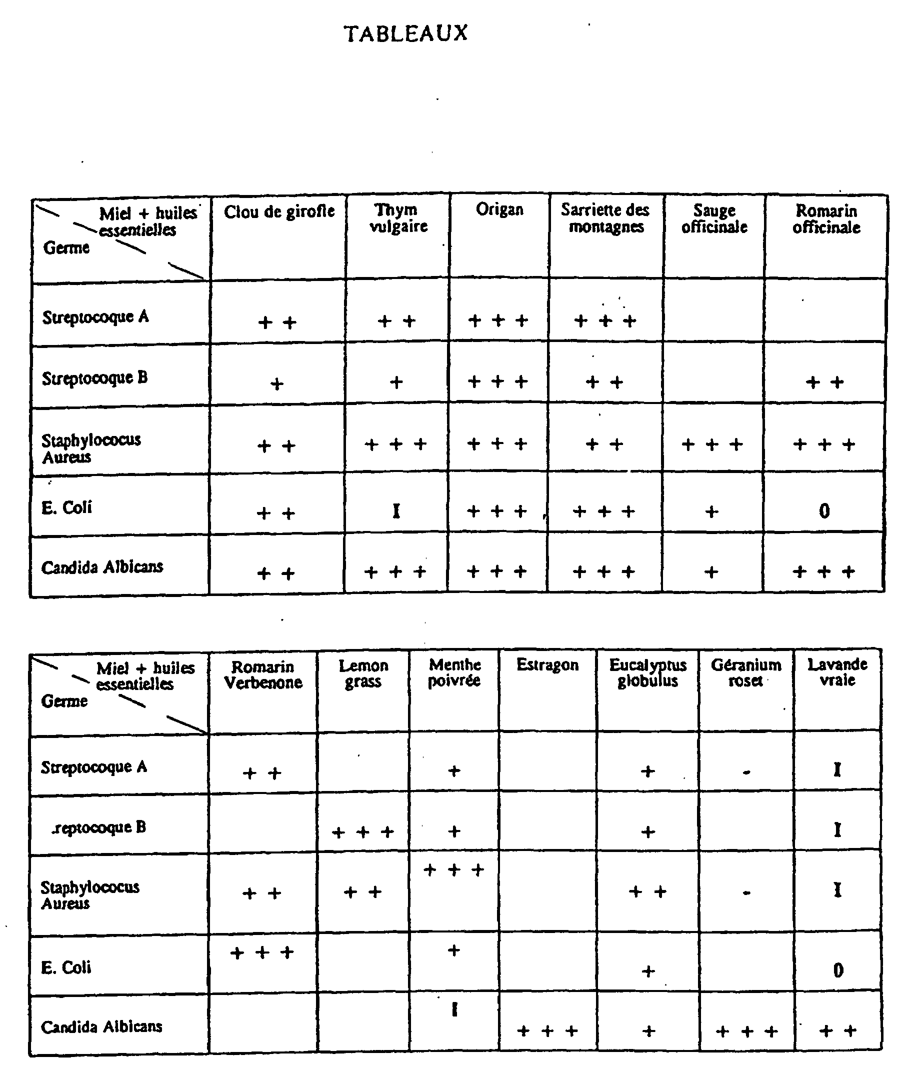

Le nombre de croix indique l'intensité de l'efficacité de la composition. Plus ce nombre est important, plus l'efficacité de la composition est grande. La lettre «I» signifie que son efficacité est moyenne et la lettre «O» nulle ou quasiment nulle. Le symbole «-» signifie qu'il y a un manque de croissance des germes mis en culture et, en présence de la composition de l'invention.

Les résultats de ce tableau montrent que la composition de l'invention comprenant de l'origan marque une très nette efficacité en présence de germes appartenant à des genres totalement différents.

## Revendications

1. Composition comprenant du miel, **caractérisée en ce qu'**elle comprend de 1 à 5 % en poids d'au moins une huile essentielle et/ou au moins un dérivé d'une huile essentielle choisie parmi le clou de girofle, la sarriette des montagnes, l'origan par rapport au poids total de la composition.

2. Composition selon la revendication 1, **caractérisée en ce que** l'huile est comprise en une quantité allant de 1 à 2 % en poids par rapport au poids total de la composition.

3. Composition selon l'une des revendications précédentes, **caractérisée en ce qu'**elle comprend en outre au moins un additif choisi parmi la propolis, les agents hydratants, les parfums, les vitamines, les oligo-éléments, les corps gras, les tensioactifs, les agents épaississants, les charges, les antioxygènes, les pigments, les gélifiants, les électrolytes, les protéines, les protides, les acides aminés, les glucides, les conservateurs, les colorants, l'eau, les alcools, la gelée royale, le pollen, les levures.

4. Composition selon la revendication 3, **caractérisée en ce qu'**elle comprend 5 % de miel, 1 % d'au moins une huile essentielle et/ou d'au moins un dérivé d'une huile essentielle, et 1 % de propolis.

5. Composition selon l'une des revendications 3 ou 4, **caractérisée en ce que** l'additif est présent en une quantité allant de 80 % à 95 % en poids par rapport au poids total de la composition, et de préférence de 85 % à 88 %.

6. Composition selon l'une des revendications précédentes, **caractérisée en ce qu'**elle se présente sous forme d'une émulsion huile dans eau ou eau dans huile, d'une solution aqueuse, d'une solution hydroalcoolique, de gel aqueux, de gel huileux, d'une dispersion de vésicules.

7. Composition selon l'une des revendications précédentes, **caractérisée en ce qu'**elle se présente sous forme de suppositoire, de comprimé, de gélule, de pommade, d'ovule, de solution, de lotion, de crème, de lait, de shampoing, de savon, de rouge à lèvre, de fard, de fond de teint, de poudre, de dentifrice, de produit solaire, de déodorant, de spray, de pansement, de sirop, de collyre, de chewing gum, de boisson, de sucette, d'ampoules.

8. Composition selon l'une des revendications précédentes, **caractérisée en ce qu'**elle consiste en une composition cosmétique et/ou de dermatologie pour la peau et/ou les muqueuses.

9. Composition selon l'une des revendications 1 à 7, **caractérisée en ce qu'**elle consiste en une composition alimentaire.

10. Composition selon l'une des revendications 1 à 7, **caractérisée en ce qu'**elle consiste en une composition destinée à la désinfection des équipements sanitaires.

## Patentansprüche

1. Zusammensetzung mit Honig, **dadurch gekennzeichnet, dass** sie mindestens 1 bis 5% Gewicht ätherisches Öl und/oder ein Derivat eines ätherisches Öls aufweist, wobei dieses zwischen der Gewürznelke, dem Gebirgsbohnenkraut und dem Origano im Verhältnis zum Gesamtgewicht der Zusammensetzung ausgewählt wird.

2. Zusammensetzung nach Anspruch 1, **dadurch gekennzeichnet, dass** das Öl eine Menge zwischen 1 bis 2% Gewicht im Verhältnis zum Gesamtgewicht der Zusammensetzung aufweist.

3. Zusammensetzung nach einem der vorstehenden Ansprüche, **dadurch gekennzeichnet, dass** sie darüber hinaus mindestens ein Additiv aufweist, wobei dieses Additiv zwischen der Propolis, den hydratierenden Agenzien, den Duftstoffen, den Vitaminen, den Spurenelementen, den Fetten, den grenzenflächenaktiven Stoffen, den Verdickungsmitteln, den Füllstoffen, den Antioxidationsmitteln, den Pigmenten, den Geliermitteln, den Elektrolyten, den Proteinen, dem Eiweiß, den Aminosäuren, den Kohlehydraten, den Konservierungsmitteln, den Farbstoffen, dem Wasser, dem Alkohol, der Gelée Royale, den Pollen, den Hefen ausgewählt wird.

4. Zusammensetzung nach Anspruch 3, **dadurch gekennzeichnet, dass** sie 5% Honig aufweist, sowie mindestens 1% ein ätherisches Öl und/oder mindestens ein Derivat von einem ätherischen Öl und 1% Propolis aufweist.

5. Zusammensetzung nach Anspruch 3, **dadurch gekennzeichnet, dass** das Additiv in einer Menge vorhanden ist, die 80% bis 95% Gewicht im Verhältnis zum Gesamtgewicht der Zusammensetzung umfasst, und vorzugsweise zwischen 85% bis 88%.

6. Zusammensetzung nach einem der vorstehenden Ansprüche, **dadurch gekennzeichnet, dass** sie eine Öl-in-Wasser-Emulsion oder Wasser-in-Öl-Emulsion, eine wässrige Lösung, eine wässerige Alkohollösung, ein wässriges Gel, ein öliges Gel, eine Blasendispersion darstellt.

7. Zusammensetzung nach einem der vorstehenden Ansprüche, **dadurch gekennzeichnet, dass** sie ein Zäpfchen, eine Tablette, eine Gelatinekapsel, eine Salbe, ein Vaginalzäpfchen, eine Lösung, eine Lotion, eine Creme, eine Milch, ein Shampoo, eine Seife, ein Lippenstift, ein Lidschatten, ein Make-up, ein Puder, eine Zahnpasta, ein Sonnenschutzmittel, ein Deodorant, ein Spray, ein Pflaster, ein Hustensaft, Augentropfen, ein Kaugummi, ein Getränk, ein Lutscher, Ampullen darstellt.

8. Zusammensetzung nach einem der vorstehenden Ansprüche, **dadurch gekennzeichnet, dass** sie eine kosmetische Zusammensetzung und/oder eine dermatologische Zusammensetzung für die Haut und/oder die Schleimhaut darstellt.

9. Zusammensetzung nach einem der Ansprüche 1 bis 7, **dadurch gekennzeichnet, dass** sie eine alimentäre Zusammensetzung darstellt.

10. Zusammensetzung nach einem der Ansprüche 1 bis 7, **dadurch gekennzeichnet, dass** sie eine Zusammensetzung darstellt, die dem Desinfizieren von sanitären Ausrüstungen dient.

## Claims

1. Composition comprising honey, **characterised in that** it comprises from 1% to 5% by weight of at least one essential oil and/or at least one essential oil derivative chosen from among clove, mountain savory and oregano.

2. Composition set forth in claim 1, **characterised in that** the oil comprises a quantity ranging from 1% to 2% by weight relative to the total weight of the composition.

3. Composition set forth in one of the preceding claims, **characterised in that** it further comprises at least one additive chosen from among propolis, hydrating agents, perfumes, vitamins, trace elements, glycerides, surfactants, thickeners, bulking agents, antioxidants, pigments, gelling agents, electrolytes, proteins, protids, amino acids, carbohydrates, preservatives, colourings, water, alcohols, royal jelly, pollen and yeasts.

4. Composition set forth in claim 3, **characterised in that** it comprises 5% honey, 1% of at least one essential oil and/or at least one essential oil extract, and 1% propolis.

5. Composition set forth in one of the claims 3 or 4, **characterised in that** the additive is present in a quantity ranging from 80% to 95% by weight relative to the total weight of the composition, and preferably from 85% to 88%.

6. Composition set forth in one of the preceding claims, **characterised in that** it is presented in the form of an oil-in-water or a water-in-oil emulsion, an aqueous solution, a aqueous alcoholic solution, an aqueous gel, an oil-based gel or a vesicle dispersion.

7. Composition set forth in one of the preceding claims, **characterised in that** it is presented in the form of a suppository, tablet, capsule, ointment, pessary, solution, lotion, cream, milk, shampoo, soap, lipstick, rouge, foundation, powder, toothpaste, sun-care product, deodorant, spray, dressing, syrup, collyrium, chewing gum, drink, lollipop or ampoule.

8. Composition set forth in one of the preceding claims, **characterised in that** it consists of a cosmetic and/or dermatological composition for the skin and/or mucous membranes.

9. Composition set forth in one of the claims 1 to 7, **characterised in that** it consists of a foodstuff.

10. Composition set forth in one of the claims 1 to 7, **characterised in that** it consists of a composition destined for use as a disinfectant for medical equipment.
